# EUROPÄISCHE PATENTSCHRIFT

(11) **EP 1 641 460 B1**
(45) Veröffentlichungstag und Bekanntmachung des Hinweises auf die Patenterteilung: **19.08.2009**
(21) Anmeldenummer: 04763030.6
(22) Anmeldetag: 28.06.2004
(51) Int. Cl.: A61K 31/519, A61K 31/191, A61P 35/00

(54) **STABILE PHARMAZEUTISCHE ZUSAMMENSETZUNGEN VON 5,10-METHYLENTETRAHYDROFOLAT**
STABLE 5, 10-METHYLENE-TETRAHYDROFOLATE PHARMACEUTICAL COMPOUNDS
COMPOSITIONS PHARMACEUTIQUES STABLES DE 5,10-METHYLENE-TETRAHYDROFOLATE

(30) Priorität: 26.06.2003 CH 20031123
(43) Veröffentlichungstag der Anmeldung: 05.04.2006
(73) Patentinhaber: Merck Eprova AG, 8200 Schaffhausen (CH)
(72) Erfinder: MOSER, Rudolf, CH-8200 Schaffhausen (CH); AMMANN, Thomas, CH-8460 Marthalen (CH)
(86) Internationale Anmeldenummer: PCT/EP2004/006944
(87) Internationale Veröffentlichungsnummer: WO 2004/112761

(56) Entgegenhaltungen:
- WO-A-95/26963
- WO-A-97/27764
- US-A- 4 931 441
- US-A- 5 434 087
- ODIN ELISABETH ET AL: "Chemical stability and human plasma pharmacokinetics of reduced folates" CANCER INVESTIGATION, Bd. 16, Nr. 7, 1998, Seiten 447-455, XP009041635 ISSN: 0735-7907

## Beschreibung

Diese Erfindung bezieht sich auf stabile pharmazeutische Zusammensetzungen von 5,10-Methylen-(6R)-, -(6S)- oder -(6R,S)-tetrahydrofolat.

Im vorliegenden Text bezieht sich der Begriff 5,10-Methylentetrahydrofolat (abgekürzt MTHF) auf 5,10-Methylentetrahydrofolsäuren und deren Polyglutamate in Form der freien Säuren, in Form von pharmazeutisch verträglichen Salzen, im Speziellen von sauren Salzen, sowie auch von Alkali- oder Erdalkalimetall-Salzen. 5,10-Methylentetrahydrofolsäuren und deren Polyglutamate beinhalten sowohl Mischungen von optischen Isomeren, insbesondere 1:1 Mischungen von Diastereoisomeren, sowie die optisch reinen Diastereoisomeren, im Speziellen optisch reine natürliche 5,1 0-Methylen-(6R)-tetrahydrofolsäure.

Pharmazeutisch verträgliche Salze können saure Salze, wie z.B. Sulfat- oder Sulfonat-Salze sein, vorzugsweise Sulfat-Salze oder können auch Alkali- oder Erdalkalimetall-Salze sein, vorzugsweise Natrium-, Kalium-, Magnesium oder Calcium-Salze.

MTHF ist ein Wirkstoff zur vorzugsweise parenteralen Anwendung in Kombination mit fluorierten Pyrimidinen wie z.B. 5-Fluoruracil (5-FU), einem weit verbreiteten Zytostatikum für die Behandlung von festen Tumoren [CoFactor Biokeys Pharmaceuticals. Seley, K.L. IDrugs 4 (1), 99-101 (2001)]. MTHF ist ein reduziertes Folat und erzielt seine chemotherapeutische Wirkung zusammen mit dem Basenanalogen 5-FdUMP durch die Hemmung des Enzyms Thymidylat Synthase (TS), welches die Umwandlung von Desoxyuridylat (dUMP) zu Desoxythymidylat (dTMP), einem zentralen Baustein für die DNS Synthese, katalysiert. Da dieser Schritt die einzige *de novo* Quelle für Desoxythymidylat in der Zelle darstellt, ist die Hemmung dieses Schlüsselenzyms durch die Verwendung von fluorierten Pyrimidinbasen, wie z.B. 5-FU oder der 5-FU Prodrug Capecitabine (Xeloda), ein Hauptansatzpunkt in der Krebstherapie. Die Inaktivierung der TS geschieht durch die Bildung eines kovalenten inhibitorischen ternären Komplexes zwischen TS, dem Basenanalogen 5-FdUMP, welches ein Metabolit von 5-FU ist, und MTHF. Eine Verstärkung der zytotoxischen Wirkung von 5-FU kann durch Erhöhung der intrazellulären Konzentration von MTHF erreicht werden, wobei dabei die Stabilität des ternären Komplexes erhöht wird. Dies bewirkt eine direkte Hemmung der DNS Synthese und Reparatur, was schliesslich zum Zelltod und Verzögerung des Tumorwachstums führt.

Die pharmazeutische Verwendung von MTHF ist beschränkt durch die äusserst hohe Empfindlichkeit gegenüber Luftoxidation [Chemical Stability and Human Plasma Pharmacokinetics of Reduced Folates. Odin, E. et al. Cancer Investigation 16 (7), 447-455 (1998), The Structure of "Active Formaldehyde" (N⁵,N¹⁰-Methylene Tetrahydrofolic Acid) Osborn, M.J. et al. J. Am. Chem. Soc. 82, 4921-4927 (1960), Folates in foods: Reactivity, stability during processing, and nutritional implications. Hawkes, J. and Villota, R. Food Sci. Nutr. 28, 439-538 (1989)]. MTHF ist ein Addukt aus Tetrahydrofolsäure (THF) und Formaldehyd [5,10-Methylene-5,6,7,8-tetrahydrofolate. Conformation of the Tetrahydropyrazine and Imidazolidine Rings. Poe, M. et al. Biochemistry 18 (24), 5527-5530 (1979), Tetrahydrofolic Acid and Formaldehyde. Kallen, R.G. Methods in Enzymology 18B, 705-716 (1971)]. In wässeriger Lösung besteht ein Gleichgewicht zwischen MTHF einerseits und THF und Formaldehyd andererseits. Zur Stabilisierung von MTHF Lösungen wurden bisher folgende Ansätze verfolgt:
Rigoroser Ausschluss von Luftsauerstoff durch Verwendung spezieller technischer Vorrichtungen für die Rekonstitution von festen Formulierungen und Injektion von MTHF in einer luftfreien Umgebung [Chemical Stability and Human Plasma Pharmacokinetics of Reduced Folates. Odin, E. et al. Cancer Investigation 16 (7), 447-455 (1998), Fluid Transfer Systems US 4,564,054].
Zugabe eines Reduktionsmittels, wie z.B. L(+)-Ascorbinsäure oder deren Salze, reduziertes γ-Glutathion, β-Mercaptoethanol, Thioglycerin, N-Acetyl-L-Cystein, etc. als Oxidationsschutz der empfindlichen MTHF und insbesondere der THF. Stabilisierung mittels Cyclodextrin-Einschlussverbindungen EP 0 579 996 (Eprova). Verwendung von hohen Konzentrationen des Wirkstoffs.

Zur Stabilisierung anderer Tetrahydrofolsäurederivate sind auch die folgenden Methoden bekannt:
Stabilisierung von Lösungen enthaltend 5-Formyltetrahydrofolsäure durch Zugabe von Natriumcitrat, Natriumacetat oder Natriumchlorid EP 0 755 396 (Pharmachemie). Stabilisierung von Injektionslösungen enthaltend ein Natrium- oder Kaliumsalz der 5-Formyltetrahydrofolsäure bei einem pH-Wert zwischen 7.5 und 8.5 EP 0 677 159 (SAPEC).
Stabilisierung von Lösungen enthaltend das Calciumsalz der 5-Formyltetrahydrofolsäure durch Zugabe von Natriumcitrat US 4,931,441 (Luitpold Pharmaceutical).

Die Stabilisierung von 5-Formyltetrahydrofolsäure, insbesondere deren Lösungen lässt sich jedoch nicht vergleichen mit der Stabilisierung von 5,10-Methylentetrahydrofolsäure Lösungen. So bewirkt die in einem fünfgliedrigen Ring eingebundene Methylengruppe in der 5,10-Methylentetrahydrofolsäure, dass im Vergleich zu 5-Formyltetrahydrofolsäure deutlich andere Substanzeigenschaften auftreten. Dies äussert sich z.B. in einem deutlich anderen Stabilitätsverhalten und anderen Abbauwegen. Im Gegensatz zu 5-Formyltetrahydrofolsäure steht 5,10-Methylentetrahydrofolsäure in Lösung immer im Gleichgewicht mit Formaldehyd und Tetrahydrofolsäure (THF), welche sich durch eine äusserst hohe Oxidationsempfindlichkeit auszeichnet. 5-Formyltetrahydrofolsäure hingegen zeigt dieses Dissoziationsverhalten nicht und ist in pharmazeutisch verträglichen wässerigen Lösungen generell sehr stabil, auch ohne Zugabe von Natriumcitrat und Natriumhydroxid.
Bis anhin wurden daher noch keine stabilen pharmazeutischen Zusammensetzungen von MTHF beschrieben.

Es wurde nun überraschend gefunden, dass die Stabilität von MTHF in wässerigen Lösungen, Suspensionen und in festen Formen, wie z.B. Pulver oder Lyophilisaten durch die Einstellung auf einen basischen pH-Wert und die gleichzeitige Verwendung von Citrat markant erhöht werden kann. Überraschenderweise erfolgt diese Stabilisierung selbst in Abwesenheit eines Reduktionsmittels.
So sind selbst ohne Zusätze von Reduktionsmitteln (Antioxidantien) und ohne Ausschluss von Luftsauerstoff MTHF Lösungen über Stunden stabil. Dies ist umso überraschender als dass bei Verwendung von Acetat, Oxalat, Maleat und anderen Säurepartnern anstelle von Citrat keine stabilen Zusammensetzungen von MTHF erhalten werden können. Dies auch im Gegensatz zur Situation bei 5-Formyltetrahydrofolsäure, wo mit Acetat ein vergleichbarer Effekt wie mit Citrat erhalten werden kann (EP 0 755 396). Citrat reduziert bei 5-Formyltetrahydrofolsäure Lösungen, die Hydrolyse und oxidative Spaltung des Grundgerüsts und vermindert somit die Bildung von Produkten wie z.B. p-Aminobenzoylglutaminsäure, Pterin- und Tetrahydropterin Derivaten. Im Gegensatz dazu hemmt Citrat im basischen pH-Bereich bei MTHF die Abspaltung von Formaldehyd (Hydrolyse) aus dem Molekül. Dies ist ein markanter und überraschender Unterschied im Verhalten dieser beiden Verbindungen, die beide der Substanzklasse der Folate angehören.
Die vorliegende Erfindung ermöglicht es überdies, selbst schwerlösliche Calcium-oder saure Salze [Eprova Patent Stabile Salze von 5,10-Methylentetrahydrofolsäure EP 0 537 492] von MTHF in hohen Konzentrationen und in physiologisch verträglichen isotonischen Lösungen zuzubereiten.

Die unerwartete Stabilisierung von MTHF mit Citrat bei basischen pH-Werten beruht auf einer überraschenden synergistischen Wirkung der Citratpufferlösung in diesem pH-Bereich. Die Komplexbildung zwischen Citrat und MTHF einerseits, sowie Citrat und dem Gegenion (Salz) von MTHF andererseits trägt massgeblich zur Stabilisierung der Methylengruppe durch Hemmung der Abspaltung von Formaldehyd (Hydrolyse) aus dem MTHF Molekül bei. Dadurch wird verhindert, dass die äusserst oxidationsempfindliche THF entsteht und MTHF abgebaut wird.

Bei den erfindungsgemässen Zusammensetzungen wird der pH-Wert im Bereich 7.5 - 10.5, vorzugsweise 8.5 - 9.5, eingestellt. Dies geschieht mit Hilfe von Natronlauge, Salzsäure in der MTHF Lösung, welche als Stabilisierungs- und Puffersubstanzen Citronensäure, Natriumdihydrogencitrat oder tri-Natriumcitrat-Dihydrat enthalten. Ein Zusatz von Reduktionsmitteln, wie z.B. L(+)-Ascorbinsäure oder deren Salze, reduziertes γ-Glutathion, β-Mercaptoethanol, Thioglycerin, N-Acetyl-L-Cystein, etc. als Oxidationsschutz ist ebenfalls möglich.

Die erfindungsgemässen Formulierungen eignen sich auch besonders zur Herstellung von Lyophilisationslösungen und Lyophilisaten oder Trockenpulvern und Trockenmischungen, da die stabilen MTHF Lösungen in hohen Konzentrationen in entsprechende Gefässe, wie z.B. Vials, Ampullen, etc., abgefüllt werden können. Die Lyophilisate sind überraschend gut lagerfähig und stabil. Durch Zugabe von Wasser oder wässerigen Lösungen lassen sie sich problemlos rekonstituieren, wobei auch die fertigen klaren Injektionslösungen wiederum ausgezeichnete Stabilitätseigenschaften aufweisen.

Bevorzugt werden die beanspruchten Formulierungen parenteral verwendet. Ebenso können aus den Lyophilisaten aber auch Formulierungen für die enterale (z. B. oral, sublingual oder rektal) oder topische (z. B. transdermale) Anwendung hergestellt werden.

Die Formulierungen werden bevorzugt direkt als wasserbasierende Lösungen oder oelbasierende Suspensionen oder als Lyophilisate verwendet. Zubereitungen für die parenterale Anwendung beinhalten sterile wässrige und nichtwässrige Injektionslösungen und Suspensionen der aktiven Verbindungen, die vorzugsweise eine isotonische Zusammensetzung aufweisen.
Formulierungen können jedoch auch mit einem Träger verabreicht werden. Als Träger können organische oder anorganische Substanzen verwendet werden, die nicht mit der aktiven Wirksubstanz reagieren, z. B. Wasser, Oel, Benzylalkohol, Polyethylenglycol, Glycerintriacetat oder andere Fettsäureglyceride, Gelatine, Lecithin, Cyclodextrine, Kohlenhydrate wie Lactobiose oder Stärke, Magnesiumstearat, Talk oder Cellulose. Bevorzugt bei der oralen Anwendung sind Tabletten, Dragées, Kapseln, Pulver, Sirup, Konzentrate oder Tropfen, für die rektale Anwendung bevorzugt sind Suppositorien.

Ebenfalls anwendbar sind Suspensionen, Emulsionen oder Implantate und für die topische Anwendung Pflaster oder Cremes.

Die Zubereitungen können Stabilisatoren, Additive für die kontrollierte Freisetzung der pharmazeutisch aktiven Verbindungen, Antioxidantien, Puffer, Bakteriostatikas und Hilfsstoffe zur Einstellung einer isotonischen Lösung beinhalten. Wässrige und nichtwässrige sterile Suspensionen können Suspensionszusatzstoffe und Verdickungsmittel beinhalten. Die Zubereitung kann als Einfachdosis- oder als Mehrfachdosis-Behälter vorhanden sein, zum Beispiel als verschweisste Ampullen oder Vials mit Stopfen und Verschlusskappe und kann als gefriergetrocknetes (lyophilisiertes) Produkt gelagert und bei Bedarf mit steriler Flüssigkeit, zum Beispiel Wasser oder physiologischer Salzlösung für den Gebrauch vorbereitet werden. In derselben Art und Weise können auch steriles Pulver, Granulat oder Tabletten verwendet werden.

Alle Zubereitungen können zusätzlich eine oder mehrere separat oder synergistisch wirkende aktive Verbindungen enthalten. Im Speziellen sind dies fluorierte Pyrimidinbasen, wie z.B. 5- Fluoruracil (5-FU), Capecitabine (Xeloda), Tegafur, UFT, Doxifluridine, 2'-Deoxy-5-fluorouridine, verschiedene Zytostatika wie z.B. Gemcitabine (Gemzar), Docetaxel (Taxotere), Paclitaxel (Taxol), Topotecan (Hycamtin), Irinotecan (CPT-11), Doxorubicin (Rubex), Mitomycin (MTC), Cisplatin (CDDP), Cyclophosphamide (CPM), Methotrexate (Amethopterin), Pemetrexed (Alimta), Vincristine (VCR), Cytarabine (Ara-C), Epirubicin (Ellence), Oxaliplatin (Eloxatin), Tamoxifen (Nolvadex), Carboplatin (CBDCA), Etoposide (Etopophos), Ifosfamide (Ifex) oder Antioxidantien, wie z.B. Vitamin C, Vitamin E, Glutathion, Thioglycerin, Acetylcystein sowie die beiden MTHF Dissoziationsprodukte Formaldehyd und Tetrahydrofolsäure.

Die Zubereitung beinhaltet zwischen 0.001 mg und 10'000 mg MTHF pro Dosis. In der Therapie werden Zubereitungen enthaltend vorzugsweise zwischen 1 mg und 1'000 mg MTHF pro Dosis eingesetzt.

Die Dosierung hängt ab von der Therapieform, von der Anwendungsform der Zubereitung, vom Alter, Gewicht, Ernährung und Zustand des Patienten. Die Behandlung kann mit tiefer Dosierung unterhalb der optimalen Menge begonnen und bis zur Erreichung des optimalen Effektes gesteigert werden. Bevorzugte Dosierungen in der Therapie bewegen sich zwischen 1 mg und 1'000 mg pro Tag, im speziellen zwischen 100 mg und 500 mg pro Tag. Die Anwendung kann entweder als Einmal-Gabe oder als wiederholte Dosierung erfolgen.

Die Zubereitungen können in allen Folatanwendungsgebieten eingesetzt werden.

Auf Basis der vorangehenden Beschreibung kann ein Fachmann auf dem Gebiet ohne Weiteres die entscheidenden Elemente der Erfindung entnehmen und ohne vom Grundgedanken und vom Umfang der Erfindung abzuweichen, Änderungen und Ergänzungen anbringen und dadurch die Erfindung an unterschiedliche Bedürfnisse und Bedingungen anpassen.

Die folgenden Beispiele können mit ähnlichem Erfolg durchgeführt werden durch Ersetzen der generisch oder spezifisch beschriebenen Produkte und/oder Verfahrensbedingungen dieser Erfindung durch solche, die in den folgenden Beispielen aufgeführt sind. Ebenso sind die folgenden spezifischen Ausführungsformen rein beispielhaft und in keiner Art und Weise limitierend auf den Rest der Offenbarung zu sehen.

### Beispiele zur illustrierung der Erfindung

### Beispiel 1

### Lyophilisat enthaltend 5,10-Methylen-(6R,S)-tetrahydrofolsäure

9900 ml Wasser werden mit Argon gesättigt. Unter Rühren werden 421.9 g Citronensäure darin vollständig gelöst. 232.0 g 5,10-Methylen-(6R,S)-tetrahydrofolsäure Calciumsalz werden zugegeben. Der pH-Wert wird mit Natronlauge auf 8.0 eingestellt, wobei sich die 5,10-Methylen-(6R,S)-tetrahydrofolsäure langsam auflöst. Danach wird der pH-Wert mit Natronlauge auf 8.5 eingestellt. Die Lösung wird steril filtriert und 5.0 ml pro Vial in 10 ml Glasvials abgefüllt. Danach wird die Lösung eingefroren und gefriergetrocknet.

Man erhält Vials enthaltend 5,10-Methylen-(6R,S)-tetrahydrofolsäure.

### Beispiel 2

### Stabilisierung von 5,10-Methylen-(6R,S)-tetrahydrofolsäure (Lyophilisat)

Nach Beispiel 1 hergestellte Vials zeigen folgende Stabilitätswerte (Am 1466-A):

**Lagerung bei +4°C (% relative Stabilität)**

| Dauer (Monate) | | | | | | | |
|---|---|---|---|---|---|---|---|
| 0 | 3.0 | 6.0 | 9.0 | 12.0 | 18.0 | 24.0 | 36.0 |
| 100.0 | 100.4 | 99.3 | 98.5 | 99.0 | 99.4 | 98.0 | |

**Lagerung bei -15°C (% relative Stabilität)**

| Dauer (Monate) | | | | | | | |
|---|---|---|---|---|---|---|---|
| 0 | 3.0 | 6.0 | 9.0 | 12.0 | 18.0 | 24.0 | 36.0 |
| 100.0 | 99.8 | 97.9 | 98.3 | 98.8 | 98.8 | 98.4 | |

Im Vergleich dazu zeigt die unbehandelte Referenzprobe des Calciumsalzes der 5,10-Methylen-(6R)-tetrahydrofolsäure folgende Stabilitätswerte (Co 751):

**Lagerung bei +25°C (% relative Stabilität)**

| Dauer (Monate) | | | | | | | |
|---|---|---|---|---|---|---|---|
| 0 | 0.5 | 1.0 | 1.75 | 2.5 | 4.5 | 9.2 | 19.0 |
| 100.0 | 92.3 | 83.4 | 77.2 | 71.9 | 65.5 | 49.1 | 43.0 |

### Beispiel 3

### Stabilisierung von 5,10-Methylen-(6R,S)-tetrahydrofolsäure (Lösungen)

Nach Beispiel 1 hergestellte Zusammensetzungen zeigen als verdünnte Lösung in physiologischer Kochsalzlösung folgende Stabilitätswerte (AC0448):

**Lagerung bei +25°C (% relative Stabilität) , ohne Luftausschluss**

| Dauer (Stunden) | | | | | | | |
|---|---|---|---|---|---|---|---|
| 0 | 0.67 | 1.33 | 2.0 | 2.67 | 3.33 | 4.0 | 32.2 |
| 100.0 | 97.6 | 95.1 | 94.6 | 93.7 | 92.1 | 89.9 | 51.8 |

Nach Beispiel 1 hergestellte Zusammensetzungen zeigen als verdünnte wässerige Lösung folgende Stabilitätswerte (AC0447):

**Lagerung bei +25°C (% relative Stabilität), ohne Luftausschluss**

| Dauer (Stunden) | | | | | | | |
|---|---|---|---|---|---|---|---|
| 0 | 0.67 | 1.33 | 2.0 | 2.67 | 3.33 | 4.0 | 23.2 |
| 100.0 | 97.7 | 97.0 | 96.6 | 94.8 | 93.8 | 93.3 | 70.9 |

Nach Beispiel 1 hergestellte Zusammensetzungen zeigen als konzentrierte wässerige Lösung folgende Stabilitätswerte (AC0447):

**Lagerung bei +25°C (% relative Stabilität) , ohne Luftausschluss**

| Dauer (Stunden) | | | | | | | |
|---|---|---|---|---|---|---|---|
| 0 | 2.0 | 4.0 | 6.0 | 12.0 | 24.0 | | |
| 100.0 | 100.2 | 99.2 | 98.1 | 95.9 | 86.2 | | |

Aus dem Stand der Technik können für das Calciumsalz der 5,10-Methylen-(6R,S)-tetrahydrofolsäure in physiologischer Kochsalzlösung als Vergleich dazu folgende Stabilitätswerte gefunden werden [siehe dazu Chemical Stability and Human Plasma Pharmacokinetics of Reduced Folates. Odin, E. et al. Cancer Investigation 16 (7), 447-455(1998)].

**Lagerung bei +25°C (% relative Stabilität)**

| | Dauer (Stunden) | | | | | | |
|---|---|---|---|---|---|---|---|
| | 0 | 4.0 | 24.0 | 26.0 | 48.0 | | |
| Mit Luftausschluss | 100.0 | 58.0 | 38.0 | 18.0 | 8.0 | | |
| Ohne Luftausschluss | 100.0 | 84 | 12.0 | 8.0 | 6.0 | | |

### Beispiel 4

### Tablette enthaltend 5,10-Methylen-(6R)-tetrahydrofolsäure

990 l Wasser werden mit Argon gesättigt. Unter Rühren werden 42.2 kg Citronensäure darin vollständig gelöst. 21.4 kg freie Säure der 5,1 0-Methylen-(6R)-tetrahydrofolsäure, werden zugegeben. Der pH-Wert wird mit Natronlauge auf 8.0 eingestellt, wobei sich die 5,10-Methylen-(6R)-tetrahydrofolsäure langsam auflöst. Danach wird der pH-Wert mit Natronlauge auf 8.5 eingestellt. Die Lösung wird steril filtriert und lyophilisiert. Eine Menge des Lyophilisates enthaltend 1'000 g 5,10-Methylen-(6R)-tetrahydrofolsäure wird mit 4 kg Lactose, 1.2 kg Stärke, 0.2 kg Talk und 0.1 kg Magnesiumstearat so zu Tabletten gepresst, dass jede Tablette 100 mg 5,10-Methylen-(6R)-tetrahydrofolsäure enthält.

Die Tablette kann auch als Filmtablette beschichtet werden.

### Beispiel 5

### Suppositorien enthaltend 5-Methylen-(6R,S)-tetrahydrofolsäure

Lyophilisat hergestellt gemäss Beispiel 1 enthaltend 500 g 5,10-Methylen-(6R,S)-tetrahydrofolsäure wird mit 50 g Hydroxypropylcellulose und 2 kg semisynthetische Glyceride so zu Suppositorien geschmolzen, dass jedes Suppositorium 500 mg 5,10-Methylen-(6R,S)-tetrahydrofolsäure enthält.

### Beispiel 6

### Kombinationspräparat enthaltend u.a. 5,10-Methylen-(6R,S)-tetrahydrofolsäure und 5-Fluoruracil

Analog zu den Beispielen 1, 4, 5 und 7 wird ein Kombinationspräparat hergestellt, enthaltend zusätzlich zur für die entsprechende Anwendung üblichen Menge an 5,10-Methylen-(6R,S)-tetrahydrofolsäure auch die für diese Anwendung übliche Menge an 5-Fluoruracil.

### Beispiel 7

### Lyophilisat enthaltend 5,10-Methylen-(6R)-tetrahydrofolsäure

9900 ml Wasser werden mit Argon gesättigt. Unter Rühren werden 316.5 g Citronensäure darin vollständig gelöst. 212.5 g 5,10-Methylen-(6R)-tetrahydrofolsäure Sulfat werden zugegeben. Der pH-Wert wird mit Natronlauge auf 8.0 eingestellt, wobei sich das 5,10-Methylen-(6R)-tetrahydrofolsäure Sulfat langsam auflöst. Danach wird der pH-Wert mit Natronlauge auf 8.5 eingestellt. Die Lösung wird steril filtriert und 5.0 ml pro Vial in 10 ml Glasvials abgefüllt. Danach wird die Lösung eingefroren und gefriergetrocknet.

Man erhält Vials enthaltend 5,10-Methylen-(6R)-tetrahydrofolsäure.

### Beispiel 8

### Stabilisierung von 5,10-Methylen-(6R)-tetrahydrofolsäure (Lösung)

Nach Beispiel 7 hergestellte Zusammensetzungen zeigen als konzentrierte wässerige Lösung folgende Stabilitätswerte (Am 1758-2/a):

**Lagerung bei +25°C (% relative Stabilität), ohne Luftausschluss**

| Dauer (Stunden) | | | | | | | |
|---|---|---|---|---|---|---|---|
| 0 | 0.67 | 1.33 | 2.67 | 4.0 | 5.33 | | |
| 100.0 | 100.2 | 99.1 | 99.2 | 98.4 | 97.7 | | |

Aus dem Stand der Technik können für das Calciumsalz der 5,10-Methylen-(6R,S)-tetrahydrofolsäure in physiologischer Kochsalzlösung als Vergleich dazu folgende Stabilitätswerte gefunden werden [siehe dazu Chemical Stability and Human Plasma Pharmacokinetics of Reduced Folates. Odin, E. et al. Cancer Investigation 16 (7), 447-455 (1998)].

**Lagerung bei +25°C (% relative Stabilität)**

| | Dauer (Stunden) | | | | | | |
|---|---|---|---|---|---|---|---|
| | 0 | 4.0 | 24.0 | 26.0 | 48.0 | | |
| Mit Luftausschluss | 100.0 | 58.0 | 38.0 | 18.0 | 8.0 | | |
| Ohne Luftausschluss | 100.0 | 84 | 12.0 | 8.0 | 6.0 | | |

## Patentansprüche

1. Stabile pharmazeutische Zusammensetzungen von 5,10-Methylen-(6R)-, -(6S)-oder -(6R,S)-tetrahydrofolat, **dadurch gekennzeichnet, dass** die Zusammensetzung 5,10-Methylen-(6R)-, -(6S)- oder -(6R,S)-tetrahydrofolsäure oder ein pharmazeutisch verträgliches Salz der 5,10-Methylen-(6R)-, -(6S)- oder -(6R,S)-tetrahydrofolsäure zusammen mit Citrat beinhaltet und einen pH-Wert zwischen 7.5 und 10.5, vorzugsweise zwischen 8.5 und 9.5 aufweist.

2. Stabile pharmazeutische Zusammensetzungen nach Anspruch 1 zusammen mit weiteren pharmazeutisch verträglichen Wirk- und Hilfsstoffen.

3. Pharmazeutische Zusammensetzung gemäss Anspruch 2 **dadurch gekennzeichnet, dass** als Hilfsstoff Formaldehyd beinhaltet ist.

4. Pharmazeutische Zusammensetzung gemäss Anspruch 2 **dadurch gekennzeichnet, dass** als weiterer Wirkstoff ein weiteres Folat beinhaltet ist.

5. Pharmazeutische Zusammensetzung gemäss Anspruch 4 **dadurch gekennzeichnet, dass** als weiteres Folat Tetrahydrofolsäure und deren Salze beinhaltet ist.

6. Pharmazeutische Zusammensetzung gemäss Anspruch 1 **dadurch gekennzeichnet, dass** als pharmazeutisch verträgliches Salz der 5,10-Methylen-(6R)-, -(6S)- oder -(6R,S)-tetrahydrofolsäure das Calciumsalz oder ein saures Salz eingesetzt wird.

7. Pharmazeutische Zusammensetzung gemäss Anspruch 2 **dadurch gekennzeichnet, dass** als weiterer Wirkstoff ein Zytostatikum beinhaltet ist.

8. Pharmazeutische Zusammensetzung gemäss Anspruch 2 **dadurch gekennzeichnet, dass** als weiterer Wirkstoff ein fluoriertes Pyrimidinderivat beinhaltet ist.

9. Pharmazeutische Zusammensetzung gemäss Anspruch 8 **dadurch gekennzeichnet, dass** als fluoriertes Pyrimidinderivat 5-Fluoruracil oder ein 5-Fluoruracil Prodrug , im Speziellen Capecitabine (Xeloda) beinhaltet ist.

10. Pharmazeutische Zusammensetzung gemäss einem der Ansprüche 1 bis 9 beinhaltend zusätzlich mindestens ein Antioxidans oder einen Radikalfänger.

11. Pharmazeutische Zusammensetzung gemäss Anspruch 10 **dadurch gekennzeichnet, dass** als Antioxidans oder Radikalfänger Vitamin C oder reduziertes Glutathion beinhaltet ist.

12. Pharmazeutische Zusammensetzung gemäss einem der Ansprüche 1 bis 11 **dadurch gekennzeichnet, dass** die Zusammensetzung als Lyophilisat, Trockenpulver oder Trockenmischung vorliegt.

13. Pharmazeutische Zusammensetzung gemäss einem der Ansprüche 1 bis 11 **dadurch gekennzeichnet, dass** die Zusammensetzung als Lyophilisationslösung vorliegt.

14. Verfahren zur Stabilisierung von Zusammensetzungen beinhaltend 5,10-Methylen-(6R)-, -(6S)- oder -(6R,S)-tetrahydrofolat, **dadurch gekennzeichnet, dass** 5,10-Methylen-(6R)-, -(6S)- oder -(6R,S)-tetrahydrofolsäure mit Citrat versetzt und auf einen pH-Wert zwischen 7.5 und 10.5, vorzugsweise zwischen 8.5 und 9.5, gebracht wird.

15. Verwendung von Zusammensetzungen beinhaltend ein pharmazeutisch verträgliches Salz der 5,10-Methylen-(6R)-, -(6S)- oder -(6R,S)-tetrahydrofolsäure und Citrat bei einem pH-Wert zwischen 7.5 und 10.5, vorzugsweise zwischen 8.5 und 9.5, zur Herstellung einer pharmazeutischen Zubereitung geeignet zum Einsatz bei entsprechenden medizinischen Indikationen.

## Claims

1. Stable pharmaceutical compositions of 5,10-methylene-(6R)-, -(6S)-or -(6R,S)-tetrahydrofolate, **characterised in that** the composition comprises 5,10-methylene-(6R)-, -(6S)- or -(6R,S)-tetrahydrofolic acid or a pharmaceutically acceptable salt of 5,10-methylene-(6R)-, -(6S)- or -(6R,S)-tetrahydrofolic acid together with citrate, and has a pH between 7.5 and 10.5, preferably between 8.5 and 9.5.

2. Stable pharmaceutical compositions according to claim 1 together with further pharmaceutically acceptable active ingredients and adjuvants.

3. A pharmaceutical composition according to claim 2, **characterised in that** it comprises formaldehyde as an adjuvant.

4. A pharmaceutical composition according to claim 2, **characterised in that** it comprises a further folate as a further active ingredient.

5. A pharmaceutical composition according to claim 4, **characterised in that** it comprises tetrahydrofolic acid and salts thereof as a further folate.

6. A pharmaceutical composition according to claim 1, **characterised in that** the calcium salt or an acidic salt is used as the pharmaceutically acceptable salt of 5,10-methylene-(6R)-, -(6S)- or -(6R,S)-tetrahydrofolic acid.

7. A pharmaceutical composition according to claim 2, **characterised in that** it comprises a cytostatic agent as a further active ingredient.

8. A pharmaceutical composition according to claim 2, **characterised in that** it comprises a fluorinated pyrimidine derivative as a further active ingredient.

9. A pharmaceutical composition according to claim 8, **characterised in that** it comprises 5-flucruracil or a 5-fluoruracil prodrug, particularly capecitabine (xeloda) as a fluorinated pyrimidine derivative,

10. A pharmaceutical composition according to any one of claims 1 to 9, additionally comprising at least one antioxidant or a radical scavenger.

11. A pharmaceutical composition according to claim 10, **characterised in that** it comprises vitamin C or reduced glutathione as an antioxidant or radical scavenger.

12. A pharmaceutical composition according to any one of claims 1 to 11, **characterised in that** the composition exists as a lyophilisate, dry powder or dry mixture.

13. A pharmaceutical composition according to any one of claims 1 to 11, **characterised in that** the composition exists as a lyophilisation solution.

14. A method of stabilising compositions comprising 5,10-methylene-(6R)-, -(6S)- or - (6R,S)-tetrahydrofolate, **characterised in that** 5,10-methylene-(6R)-, -(6S)- or -(6R,S)-tetrahydrofolic acid is treated with citrate and is brought to a pH between 7.5 and 10.5, preferably between 8.5 and 9.5.

15. Use of compositions comprising a pharmaceutically acceptable salt of 5,10-methylene-(6R)-, -(6S)- or -(6R,S)-tetrahydrofolic acid and citrate at a pH between 7.5 and 10.5, preferably between 8.5 and 9.5, for producing a pharmaceutical preparation suitable for use for corresponding medical indications.

## Revendications

1. Compositions pharmaceutiques stables de 5,10-méthylène-(6R)-, -(6S)- ou -(6R,S)-tétrahydrofolate, **caractérisées en ce que** la composition comprend de l'acide 5,10-méthylène-(6R)-, -(6S)-ou -(6R,S)-tétrahydrofolique ou un sel pharmaceutiquement acceptable de l'acide 5,10-méthylène-(6R)-, -(6S)- ou -(6R,S)-tétrahydrofolique conjointement avec du citrate et présente une valeur de pH située entre 7,5 et 10,5, de préférence entre 8,5 et 9,5.

2. Compositions pharmaceutiques stables selon la revendication 1 conjointement avec d'autres principes actifs et auxiliaires pharmaceutiquement acceptables.

3. Composition pharmaceutique selon la revendication 2, **caractérisée en ce que** du formaldéhyde est présent à titre d'auxiliaire.

4. Composition pharmaceutique selon la revendication 2, **caractérisée en ce qu'**un autre folate est présent en tant qu'autre principe actif.

5. Composition pharmaceutique selon la revendication 4, **caractérisée en ce que** de l'acide tétrahydrofolique et ses sels sont présents en tant qu'autre folate.

6. Composition pharmaceutique selon la revendication 1, **caractérisée en ce que** l'on met en oeuvre le sel calcique ou un sel acide à titre de sel pharmaceutiquement acceptable de l'acide 5,10-méthylène-(6R)-, -(6S)- ou -(6R,S)-tétrahydrofolique.

7. Composition pharmaceutique selon la revendication 2, **caractérisée en ce qu'**un cytostatique est présent en tant qu'autre principe actif.

8. Composition pharmaceutique selon la revendication 2, **caractérisée en ce qu'**un dérivé fluoré de pyrimidine est présent en tant qu'autre principe actif.

9. Composition pharmaceutique selon la revendication 8, **caractérisée en ce que** du 5-fluoruracil ou un promédicament de 5-fluoruracil, en particulier la capécitabine (Xeloda), est présent à titre de dérivé fluoré de pyrimidine.

10. Composition pharmaceutique selon l'une des revendications 1 à 9, comprenant en plus au moins un antioxydant ou un capteur de radicaux libres.

11. Composition pharmaceutique selon la revendication 10, **caractérisée en ce que** de la vitamine C ou un glutathion réduit est présent à titre d'antioxydant ou de capteur de radicaux libres.

12. Composition pharmaceutique selon l'une des revendications 1 à 11, **caractérisée en ce que** la composition se présente sous forme de lyophilisat, de poudre sèche ou de mélange sec.

13. Composition pharmaceutique selon l'une des revendications 1 à 11, **caractérisée en ce que** la composition se présente sous forme de solution de lyophilisation.

14. Procédé de stabilisation des compositions comprenant du 5,10-méthylène-(6R)-, -(6S)- ou -(6R, S)-tétrahydrofolate, **caractérisé en ce que** l'acide 5,10-méthylène-(6R)-, -(6S)- ou -(6R,S)-tétrahydrofolique est mélangé au citrate et porté à une valeur de pH située entre 7,5 et 10,5, de préférence entre 8,5 et 9,5.

15. Utilisation de compositions comprenant un sel pharmaceutiquement acceptable de l'acide 5,10-méthylène-(6R)-, -(6S)- ou -(6R,S)-tétrahydrofolique et du citrate à une valeur de pH située entre 7,5 et 10,5, de préférence entre 8,5 et 9,5 pour la préparation d'une composition pharmaceutique appropriée à la mise en oeuvre dans des indications médicales correspondantes.
